# EUROPÄISCHE PATENTSCHRIFT

(11) **EP 1 184 042 B1**
(45) Veröffentlichungstag und Bekanntmachung des Hinweises auf die Patenterteilung: **26.07.2006**
(21) Anmeldenummer: 00118590.9
(22) Anmeldetag: 28.08.2000
(51) Int. Cl.: A61M 1/00

(54) **Absaugpumpe**
Suction pump
Pompe d'aspiration

(43) Veröffentlichungstag der Anmeldung: 06.03.2002
(73) Patentinhaber: Medela AG, 6340 Baar (CH)
(72) Erfinder: Greter, Andy, 6312 Steinhausen (CH); Kuenzler, Hansruedi, 8932 Mettmenstetten (CH); Silver, Brian, Cary, IL 60013 (US)
(74) Vertreter: Clerc, Natalia

(56) Entgegenhaltungen:
- WO-A-00/15277
- US-A- 3 084 691
- US-A- 5 419 687
- US-A- 5 466 229
- US-A- 5 662 627

## Beschreibung

Die vorliegende Erfindung betrifft eine Absaugpumpe für medizinische Zwecke, insbesondere Sekretabsaugpumpe, mit einem Auffangbehälter für abzusaugendes Material, einem Ventilblock zur wechselweisen Verbindung des Pumpenraumes über entsprechende Ventile mit dem Auffangbehälter zwecks Aufbau eines Vakuums in letzterem bzw. mit einer Ausblasleitung, je nach der Bewegungsrichtung eines Membranpumpenstössels, und einer Antriebseinheit des Membranpumpenstössels.

Die heute bekannten Absaugpumpen dieser Art haben den Nachteil, dass mit Ausnahme des abnehmbaren Auffangbehälters die Reinigung der übrigen Pumpenteile aufwendig und schwierig ist, da insbesondere grosse Sorgfalt anzuwenden ist, um das Eindringen von Flüssigkeit (Waschflüssigkeit, Sekretreste) in die Antriebseinheit zu vermeiden.

Eine solche Pumpe ist aus der US-A-5419687 bekannt. Dort ist eine Pumpe offenbart mit einem Gehäuse, in welchem Antriebseinheit und Ventilanordnungen vorhanden sind. Ein Auffangbehälter kann lösbar daran angekoppelt werden.

Aufgabe der vorliegenden Erfindung war die Schaffung einer Absaugpumpe, welche bei möglichst einfachem Aufbau leicht zusammenbaubar und für Reinigungszwecke wieder ohne besondere Kenntnisse auseinandernehmbar ist (keine Schlauchverbindungen). Die Bedienung soll äusserst einfach sein (ein Knopf Ein/Aus) und der Auffangbehälter nach seiner Entleerung in der Waschmaschine, z.B. Geschirrwaschmaschine waschbar sein. Auch die übrigen Pumpenteile, d.h. Ventilblock und Antriebseinheit, welche dank einer besonderen Ueberlaufkonstruktion des Auffangbehälters keiner Verschmutzung ausgesetzt sind, sollen bei Bedarf ohne Beeinträchtigung von deren Funktionalität, gereinigt werden können.

Diese Aufgabe wird bei einer Absaugpumpe der eingangs definierten Art erfindungsgemäss durch die Merkmale des kennzeichnenden Teils von Anspruch 1 gelöst.

Vorzugsweise werden dabei die verschiedenen Pumpenteile auf einer gemeinsamen Grundplatte mittels Steck- und/oder Schnappverbindungen lösbar zusammengefügt, angeordnet.

Die Erfindung wird nachstehend anhand eines in der Zeichnung dargestellten Ausführungsbeispiels noch etwas näher erläutert.

Es zeigt:
- Fig. 1: rein schematisch eine aus drei Hauptteilen zu bildende Absaugpumpe;
- Fig. 2: eine aus den drei Hauptteilen gemäss Fig. 1 zusammengesetzte Absaugpumpe; und
- Fig. 3: einen Längsschnitt durch die Absaugpumpe nach Fig. 2 in etwas vergrössertem Massstab.

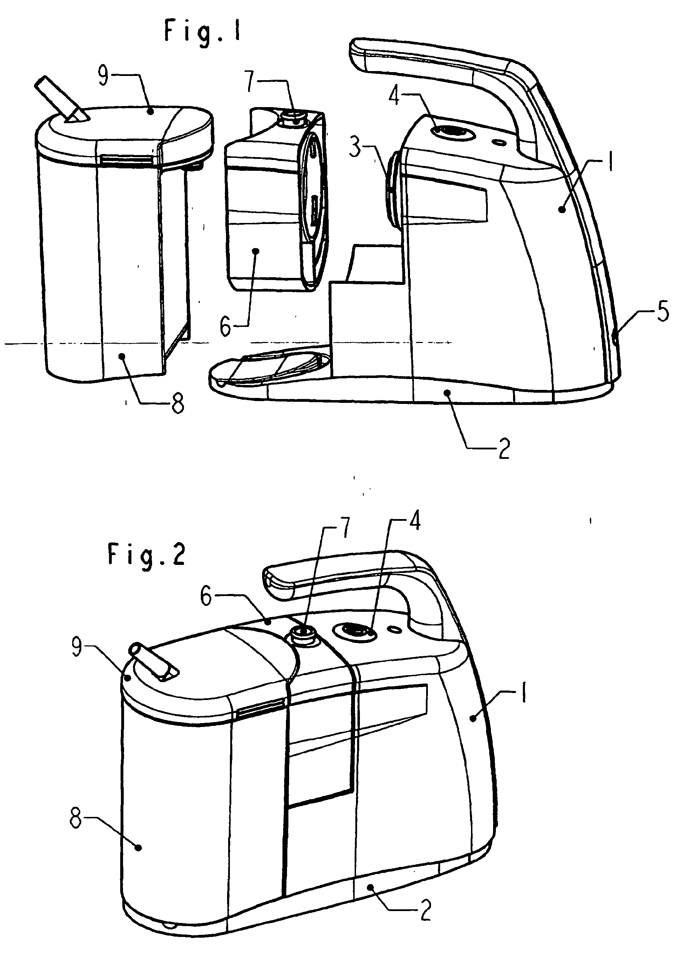

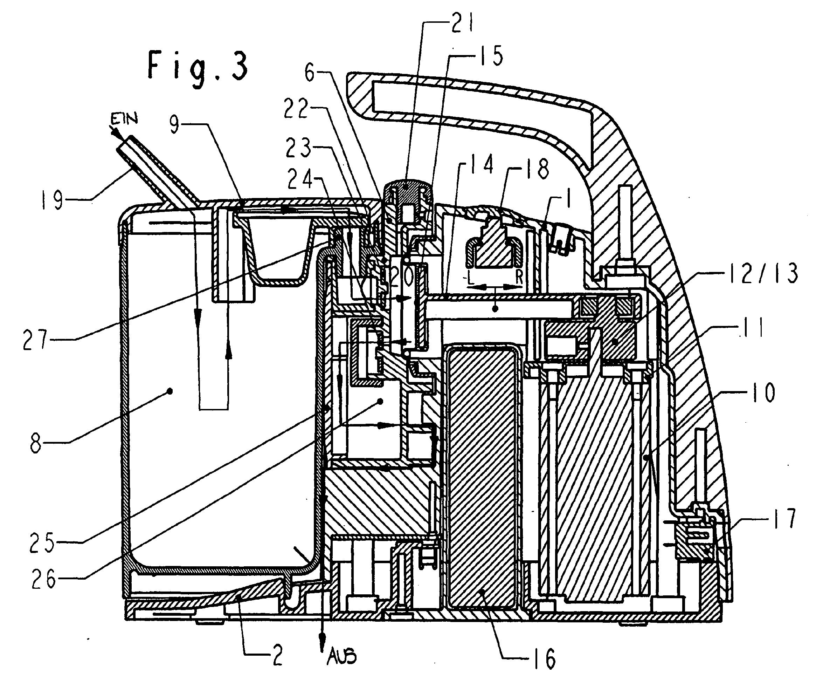

Figur 1 der Zeichnung zeigt die drei Hauptteile der Absaugpumpe, nämlich das Hauptgehäuse 1 mit eingebautem Motor, Pumpenantrieb und gegebenenfalls einem Akku (diese Einbauteile sind in Fig. 3 dargestellt), welches auf eine Bodenplatte 2 angesteckt ist, wobei am Hauptgehäuse noch die Membran 3 eines Membranpumpenstössels (s. Fig. 3), der Hauptschalter 4 (Ein/Aus) sowie ein Stromanschluss 5 gezeigt sind, ferner ein Ventilblock 6 mit Ueberlaufkammer (Fig. 3) und Vakuumeinstellknopf 7 (z.B. zwei Stellungen) und schliesslich ein wiederverwendbarer Auffangbehälter 8 mit aufgesetztem Deckel 9.

Die in Figur 1 gezeigten Hauptteile der Absaugpumpe lassen sich leicht zusammenstecken (Steck- und Schnappverbindungen) und selbstverständlich ebenso leicht wieder auseinandernehmen (zu Reinigungszwecken). Der Ventilblock 6 kommt dabei direkt zwischen das Hauptgehäuse 1 und den auswechselbaren Auffangbehälter 8 zu liegen, wie Figur 2 zeigt. Die Hauptteile sind vorzugsweise auf die Bodenplatte 2 aufgesteckt.

Das erfindungsgemässe Aufbaukonzept ist am besten aus dem Längsschnitt gemäss Figur 3 ersichtlich:

Im Hauptgehäuse 1 ist ein Elektromotor 10 (z.B. ein 12V-Gleichstrommotor) an einer Platte 11 angeflanscht und dient dem Antrieb des Exzenters 12 (mit zusätzlicher Unwucht 13). Der Exzenter 12 dient dem Antrieb des Pumpenpleuels 14, welcher mit einer Membrane 15 (z.B. umspritzte Membrane) versehen ist und im Betrieb der Erzeugung eines gewünschten Vakuums dient. Ebenfalls im Hauptgehäuse 1, welches auf eine Bodenplatte 2 aufgesteckt ist, ist ein Akku 16 untergebracht. Eine Stromversorgung von aussen erfolgt über die Anschlussbuchse 17 (12V-Gleichstrom zur Speisung des Akku 16 oder zur direkten Speisung des Motors 10). Auf der Oberseite des Gehäuses 1 ist schliesslich ein Druckknopfschalter 18 (Ein/Aus) angeordnet.

Ebenfalls auf die Grundplatte 2 aufgesteckt befindet sich auf der linken Seite der austauschbare, wiederverwendbare Auffangbehälter 8 für abzusaugendes Material (z.B. Sekret) mit Behälterdeckel 9. Das durch im Behälter 8 erzeugtes Vakuum abzusaugende Material tritt über eine am Stutzen 19 anbringbare Schlauchleitung (nicht dargestellt) in den Behälter 8 ein.

Zwischen Auffangbehälter 8 und Hauptgehäuse 1 ist als dritter separater Hauptteil ein Ventilblock 6 eingesetzt, wobei dieser durch geeignete Verbindungen mit dem Behälter 8 und dem Gehäuse 1 lösbar gekuppelt ist. Da zwischen Ventilblock 6 und dem im Hauptgehäuse 1 untergebrachten Membranpumpenstössel 14 mit an seinem vorderen Ende angeordneter Membran 15 der eigentliche Pumpenraum 20 liegt, ist dieser Raum mit einer Einrichtung zur Einstellung des gewünschten Vakuums (Vakuumeinstellknopf 21 mit z.B. zwei unterschiedlichen Stellungen) verbunden.

In einer Wand 22 des Ventilblocks 6 sind selbsttätig wirkende Ventile 23 bzw. 24 angeordnet: Bei sich in Richtung R bewegendem Pleuel 14 bzw. Membrane 15 entsteht Unterdruck und Luft Wird aus dem Behälter 8 über das sich öffnende Ventil 23 gesaugt (zwecks Erzeugung des Vakuums im Auffangbehälter). Bei der Stossbewegung des Pleuels 14 bzw. der Membran 15 in Richtung L schliesst das Ventil 23 und die in den Pumpenraum 20 angesaugte Luft wird über das sich öffnende Ventil 24 ausgestossen, dies in einen mittels eines Deckels 25 geschlossenen Raum 26, welcher über freie Kanäle zwischen den miteinander gekuppelten Pumpenteilen mit der Umgebung verbunden ist (Aus).

Die Verbindung zwischen Auffangbehälter 8 und Ventilblock 6 erfolgt über einen hochgelegenen Stutzen 27, welcher notfalls auch einen Ueberlauf für den Behälter 8 bildet. Dank der besonderen Konstruktion würde eventuell in den Ventilblock 6 eindringende Flüssigkeit die Antriebseinheit der Pumpe nicht beeinträchtigen.

Die einzelnen Pumpenteile 1, 6 und 8 können auf einfachste Weise zusammengebaut und demontiert werden, was insbesondere deren Reinigung vereinfacht und problemlos gestaltet (Auffangbehälter 8 und Ventilblock können in der Geschirrwaschmaschine gereinigt werden).

## Patentansprüche

1. Absaugpumpe für medizinische Zwecke, insbesondere Sekretabsaugpumpe, mit einem Auffangbehälter (8) für abzusaugendes Material, und einer Antriebseinheit (1,10-18) des Pumpenpleuels (14),
**dadurch gekennzeichnet, dass**
ein Ventilblock (6) mit einer mit Ventilen (23,24) versehenen Wand zur wechselweisen Verbindung des Pumpenraumes (20) über entsprechende Ventile (23,24) mit dem Auffangbehälter (8) zwecks Aufbau eines Vakuums in letzterem bzw. mit einer Ausblasleitung, je nach der Bewegungsrichtung eines Pumpenpleuels (14) angeordnet ist,
wobei Auffangbehälter (8), Ventilblock (6) und Antriebseinheit (1,10-18) als separate Pumpenteile ausgebildet sind, welche für den Betrieb der Pumpe lösbar miteinander kuppelbar sind,
wobei der Ventilblock (6) direkt zwischen Auffangbehälter (8) und Antriebseinheit (1,10-18) zu liegen kommt,
wobei der Pumpenraum (20) zwischen Ventilblock (6) und Antriebseinheit (1,10-18) liegt und durch die mit den Ventilen (23,24) versehene Wand des Ventilblocks (6) und eine Wand der Antriebseinheit (1,10-18) bzw. eine Membran (15) definiert ist und
wobei der in der Antriebseinheit (1,10-18) angeordnete Pumpenpleuel (14) an seinem vorderen Ende mit der Membran (15) zusammenwirkt, welche gegenüber dem Inneren der Antriebseinheit (1,10-18) dichtend angeordnet ist, nach aussen zum Pumpenraum (20) jedoch frei liegt.

2. Absaugpumpe nach Anspruch 1, **dadurch gekennzeichnet, dass** die lösbar miteinander zusammengefügten Pumpenteile (1,10-18;6;8)auf einer gemeinsamen Grundplatte (2) angeordnet sind.

3. Absaugpumpe nach Anspruch 1 oder 2, **dadurch gekennzeichnet, dass** die lösbare Verbindung zwischen den Pumpenteilen (1,10-18;6;8) über Steck- und/oder Schnappverbindungen erfolgt.

4. Absaugpumpe nach einem der Ansprüche 1 bis 3, **dadurch gekennzeichnet, dass** der Ventilblock (6) im eingebauten Zustand mit zwei voneinander getrennten Abteilen kommuniziert, nämlich einem ersten Abteil, welches mit dem Auffangbehälter (8) einerseits und über ein Ansaugventil (23) mit dem Pumpenraum (20) andererseits verbunden werden kann, sowie einem zweiten Abteil (26), welches über ein Auslassventil (24) den Pumpenraum (20) mit der Ausblasleitung verbindet.

5. Absaugpumpe nach einem der vorhergehenden Ansprüche, **dadurch gekennzeichnet, dass** die Pumpenteile (1,10-18;6;8) leicht zusammenbaubar und für Reinigungszwecke wieder ohne besondere Kenntnisse auseinandernehmbar sind, wobei keine Schlauchverbindungen vorhanden sind.

6. Absaugpumpe nach einem der vorhergehenden Ansprüche, **dadurch gekennzeichnet, dass** die Antriebseinheit (1,10-18) ein Hauptgehäuse (1), einen Pumpenantrieb (10-13) und gegebenenfalls einen Akku (16) aufweist, wobei das Hauptgehäuse (1) auf einer Bodenplatte (2) aufgesteckt ist.

7. Absaugpumpe nach einem der vorhergehenden Ansprüche, **dadurch gekennzeichnet, dass** der Ventilblock (6) einen Vakuumeinstellknopf (7,21) aufweist, welcher zur Einstellung des gewünschten Vakuums mit dem Pumpenraum (20) verbunden ist.

## Claims

1. Suction pump for medical applications, particularly pump for sucking off secretions, with a collection container (8) for material to be sucked off and a driving unit (1, 10-18) of the pump plunger (14),
**characterized in that**
a valve block (6) with a wall having valves (23, 24) for the alternate connection of the pump chamber (20) to respective valves (23, 24) with the collection container (8) for providing a vacuum in latter or with a blowing off circuit respectively, is arranged depending on the direction of movement of a pump plunge (14),
whereby a collection container (8), valve block (6) and driving unit (1, 10-18) are designed as separate pump parts which for the operation of the pump can be releasable coupled to each other,
whereby the valve block (6) comes to lie directly between collecting container and driving unit (1, 10-18),
whereby, the pump chamber (20) lies between valve block (6) and driving unit (1, 10-18) and is defined by the wall of the valve block (6) with the valves (23, 24) and a wall of the driving unit (1, 10-18) or a membrane (15) respectively, and
whereby the pump plunger (14) arranged in the driving unit (1, 10-18) cooperates at its front end with the membrane (15), which is arranged sealed against the inside of the driving unit (1, 10-18), is however exposed towards the environment to the pump chamber (20).

2. Suction pump according claim 1, **characterized in that** the releasable joined pump parts (1, 10-18; 6; 8) are arranged on a common plate (2).

3. Suction pump according claim 1 or 2, **characterized in that** the releasable connection between the pump parts (1, 10-18; 6; 8) proceeds through plug in and/or snap on connections.

4. Suction pump according to one of the claims 1 to 3, **characterized in that** the valve block (6) communicates in the assembled state with two compartments separated from each other, namely a first compartment, which on the one hand can be connected to the collecting container (8) and on the other hand can be connected through a suction valve (23) to the pump chamber (20), as well as a second compartment (26), which connects the pump chamber (20) through an outlet valve (24) to a blowing off circuit.

5. Suction pump according to any of the preceding claims, **characterized in that** the pump parts (1, 10-18; 6; 8) are easily mountable and for cleaning purposes easily detachable again, without outstanding knowledge, whereby no hose connections are present.

6. Suction pump according to any of the preceding claims, **characterized in that** the driving unit (1, 10-18) has a main housing (1), a pump drive (10-13) and optionally an accumulator (16), whereby the main housing (1) is mounted on a support plate (2).

7. Suction pump according to any one of the preceding claims, **characterized in that** the valve block (6) has a vacuum adjusting button (7, 21), which is connected to the pump chamber (20) for adjustment of the desired vacuum.

## Revendications

1. Pompe d'aspiration à usage médical, notamment pompe d'aspiration de sécrétions, comprenant un récipient collecteur (8) pour la matière aspirée, et une unité d'entraînement (1, 10-18) de la bielle de pompe (14),
**caractérisée en ce que**
l'on prévoit un bloc-soupapes (6) avec une paroi pourvue de soupapes (23, 24) pour la connexion en alternance de l'espace de la pompe (20) par le biais de soupapes correspondantes (23, 24) au récipient collecteur (8) dans le but de créer un vide dans ce dernier ou avec une conduite d'évacuation, en fonction du sens de déplacement d'une bielle de pompe (14),
le récipient collecteur (8), le bloc-soupapes (6) et l'unité d'entraînement (1, 10-18) étant réalisés sous forme de parties de pompe séparées, qui peuvent être accouplées de manière amovible les unes aux autres pour le fonctionnement de la pompe,
le bloc-soupapes (6) venant directement se positionner entre le récipient collecteur (8) et l'unité d'entraînement (1, 10-18),
l'espace de la pompe (20) se trouvant entre le bloc-soupapes (6) et l'unité d'entraînement (1, 10-18) et étant défini par la paroi du bloc-soupapes (6) pourvue des soupapes (23, 24) et par une paroi de l'unité d'entraînement (1, 10-18) ou par une membrane (15), et
la bielle de pompe (14) disposée dans l'unité d'entraînement (1, 10-18) coopérant à son extrémité avant avec la membrane (15), qui est disposée de manière hermétique par rapport à l'intérieur de l'unité d'entraînement (1, 10-18), mais qui est découverte vers l'extérieur vers l'espace de la pompe (20).

2. Pompe d'aspiration selon la revendication 1, **caractérisée en ce que** les parties de pompe assemblées les unes aux autres de manière amovible (1, 10-18 ; 6 ; 8) sont disposées sur une plaque, de base commune (2).

3. Pompe d'aspiration selon la revendication 1 ou 2, **caractérisée en ce que** la connexion amovible entre les parties de la pompe (1, 10-18 ; 6 ; 8) s'effectue par le biais de connexions par enfichage et/ou par encliquetage.

4. Pompe d'aspiration selon l'une quelconque des revendications 1 à 3, **caractérisée en ce que** le bloc-soupapes (6) communique dans l'état monté avec deux compartiments séparés l'un de l'autre, à savoir un premier compartiment qui peut être connecté d'une part au récipient collecteur (8) et d'autre part à l'espace de la pompe (20) par le biais d'une soupape d'aspiration (23), ainsi qu'un deuxième compartiment (26) qui relie l'espace de la pompe (20) à la conduite d'évacuation par le biais d'une soupape de sortie (24).

5. Pompe d'aspiration selon l'une quelconque des revendications précédentes, **caractérisée en ce que** les parties de la pompe (1, 10-18 ; 6 ; 8) sont faciles à assembler les unes aux autres et peuvent être démontées à nouveau sans connaissances particulières à des fins de nettoyage, aucun raccord pour tuyau flexible n'étant prévu.

6. Pompe d'aspiration selon l'une quelconque des revendications précédentes, **caractérisée en ce que** l'unité d'entraînement (1, 10-18) présente un boîtier principal (1), un entraînement de pompe (10-13) et éventuellement un accumulateur (16), le boîtier principal (1) étant posé sur une plaque de fond (2).

7. Pompe d'aspiration selon l'une quelconque des revendications précédentes, **caractérisée en ce que** le bloc-soupapes (6) présente une tête d'ajustage du vide (7, 21), qui est connectée à l'espace de la pompe (20) pour ajuster le vide souhaité.
